(19) Europäisches Patentamt
European Patent Office
Office européen des brevets

(11) Veröffentlichungsnummer: **0 130 461**
**A2**

(12) **EUROPÄISCHE PATENTANMELDUNG**

(21) Anmeldenummer: **84107002.2**

(22) Anmeldetag: **19.06.84**

(51) Int. Cl.⁴: **C 07 D 471/04,** C 07 D 487/04, C 07 D 473/28, C 07 D 473/30, C 07 D 473/04 // A61K31/44, A61K31/495, A61K31/50, A61K31/52 ,(C07D471/04, 235/00, 221/00),(C07D487/04, 237/00, 235/00),(C07D487/04, 241/00, 235/00),(C07D487/04, 253/00, 235/00)

(30) Priorität: **05.07.83 DE 3324115**

(43) Veröffentlichungstag der Anmeldung: **09.01.85 Patentblatt 85/2**

(84) Benannte Vertragsstaaten: **AT BE CH DE FR GB IT LI LU NL SE**

(71) Anmelder: **Dr. Karl Thomae GmbH, Postfach 1755, D-7950 Biberach (Riss) (DE)**

(72) Erfinder: **Austel, Volkhard, Dr. Dipl.-Chem., Kapellenweg 7, D-7950 Biberach 1 (DE)**
Erfinder: **Heider, Joachim, Dr. Dipl.-Chem., Am Hang 3, D-7951 Warthausen 1 (DE)**
Erfinder: **Hauel, Norbert, Dr. Dipl.-Chem., Händelstrasse 12, D-7950 Biberach 1 (DE)**
Erfinder: **Reiffen, Manfred, Dr. Dipl.-Chem., Matthias-Erzberger-Strasse 40, D-7950 Biberach 1 (DE)**
Erfinder: **van Meel, Jacobus C. A. Dr., Amriswilstrasse 7, D-7950 Biberach 1 (DE)**
Erfinder: **Diederen, Willi, Dr., Haldenstrasse 1a, D-7950 Biberach 1 (DE)**

(54) **Neue Imidazole, ihre Herstellung und diese Verbindungen enthaltende Arzneimittel.**

(57) Die Erfindung betrifft neue Imidazole der allgemeinen Formel

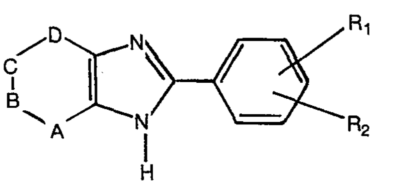

, (I)

in der
A, B, C und D je eine N-, HN-, $R_3N$-, O=C-, HC-, HO-C- oder $R_3SO_2$-O-C-Gruppe, wobei mindestens eine der Gruppen A, B, C oder D eine HN-Gruppe und eine andere der Gruppen A, B, C oder D eine O=C-Gruppe oder
mindestens eine der Gruppen A, B, C oder D ein N-Atom und eine andere der Gruppen A, B, C oder D eine HO-C-, $R_3$O-C- oder eine $R_3SO_2$-O-C-Gruppe darstellen muß
oder auch einer der Reste A, B, C oder D eine Chlormethingruppe, wenn mindestens zwei der Reste A, B, C oder D ein N-Atom darstellen,
$R^1$ eine NC-$CH_2$-O-, HOOC-$CH_2$-O-, $R_3$-OOC-$CH_2$-O-, $R_3SO_2$-O-, $R_3$-$SO_2$-NH-, $R_3$-$SO_2$-$NR_3$ oder $R_4$-O-Gruppe oder, wenn A, B, C und D zusammen mit dem ankondensierten Imidazolring kein Imidazo[4,5-c]pyridin darstellen, eine Hydroxygruppe und
$R_2$ eine $R_3$O-, 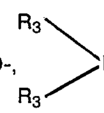 N- oder $R_5$-O-Gruppe,
wobei $R_3$ jeweils eine Alkylgruppe und
$R_4$ eine Alkinylgruppe oder, wenn A, B, C und D zusammen mit dem ankondensierten Imidazolring kein Imidazo[4,5-c]pyridin bilden, auch eine Alkenylgruppe und
$R_5$ eine Alkenyl- oder Alkinylgruppe darstellen, bedeuten, deren Tautomere und deren Säureadditionssalze, insbesondere deren physiologisch wertvolle pharmakologische Eigenschaften aufweisen, insbesondere eine Wirkung auf die Kontraktilität des Herzmuskels.

Die neuen Verbindungen lassen sich nach für analoge Verbindungen üblichen Verfahren herstellen.

EP 0 130 461 A2

0130461

DR. KARL THOMAE GMBH
D-7950 Biberach 1

Case 5/885
Dr.Fl./Kp.

# Neue Imidazole, ihre Herstellung und diese Verbindungen enthaltende Arzneimittel

In der EP-A-0.072 926 und in der EP-A-0.079.083 werden bereits Imidazo[4,5-c]pyridine beschrieben, welche wertvolle pharmakologische Eigenschaften aufweisen.

Es wurde nun gefunden, daß die neuen Imidazole der allgemeinen Formel

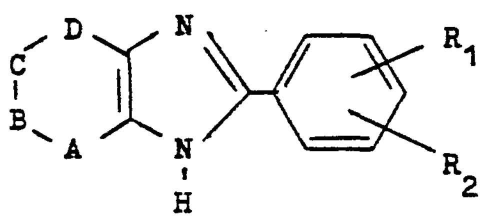

,(I)

deren Tautomere und deren Säureadditonssalze, insbesondere deren physiologisch verträgliche Säureadditionssalze mit anorganischen oder organischen Säuren, welche sich von den bereits bekannten Imidazolderivaten entweder durch den Substituenten $R_1$ oder durch die Reste A, B, C und D unterscheiden, überlegene pharmakologische Eigenschaften aufweisen, insbesondere eine Wirkung auf die Kontraktilität des Herzmuskels.

In der obigen allgemeinen Formel I bedeutet

A, B, C und D je eine N-, HN-, $R_3N$-, O=C-, HC-, HO-C- oder $R_3SO_2$-O-C-Gruppe, wobei mindestens eine der Gruppen A, B, C oder D eine HN-Gruppe und eine andere der Gruppen A, B, C oder D eine O=C-Gruppe oder mindestens eine der Gruppen A, B, C oder D ein N-Atom und eine andere der Gruppen A, B, C oder D eine HO-C-, $R_3$0-C- oder eine $R_3SO_2$-O-C-Gruppe darstellen muß, oder auch einer der Reste A, B, C oder D eine Chlormethingruppe, wenn mindestens zwei der Reste A, B, C oder D ein N-Atom darstellen,

$R_1$ eine NC-$CH_2$-O-, HOOC-$CH_2$-O-, $R_3$-OOC-$CH_2$-O-, $R_3SO_2$-O-, $R_3$-$SO_2$-NH-, $R_3$-$SO_2$-$NR_3$- oder $R_4$-O-Gruppe oder, wenn A, B, C und D zusammen mit dem ankondensierten Imidazolring kein Imidazo[4,5-c]pyridin darstellen, eine Hydroxygruppe und

$R_2$ eine $R_3$O-, 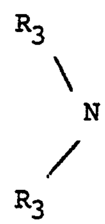 - oder $R_5$-O-Gruppe,

wobei $R_3$ jeweils eine Alkylgruppe mit 1 bis 4 Kohlenstoffatomen und

$R_4$ eine Alkinylgruppe mit 2 bis 5 Kohlenstoffatomen oder, wenn A, B, C und D zusammen mit dem ankondensierten Imidazolring kein Imidazo[4,5-c]pyridin bilden, auch eine Alkenylgruppe mit 2 bis 5 Kohlenstoffatomen und

$R_5$ eine Alkenyl- oder Alkinylgruppe mit jeweils 2 bis 5 Kohlenstoffatomen darstellen.

Gegenstand der vorliegenden Erfindung sind somit insbesondere die neuen Imidazo[4,5-b]pyridin-5-one, Imidazo[4,5-b]-pyridin-7-one, 6-Hydroxy-imidazo[4,5-b]pyridine, Imidazo-[4,5-c]pyridin-6-one, Imidazo[4,5-c]pyridin-4-one, 7-Hydroxy-imidazo[4,5-c]pyridine, Imidazo[4,5-c]pyridazin-6-one, Imidazo[4,5-d]pyridazin-4-one, Imidazo[4,5-b]pyrazin-5-one, Purin-2-one, Purin-6-one, Purin-2,6-dione, Imidazo[4,5-e]-triazin-6-one, Imidazo[4,5-d]triazin-7-one, Alkansulfonyloxy-imidazo[4,5-b]pyridine, Alkansulfonyloxy-imidazo[4,5-c]-pyridine, Alkansulfonyloxy-purine, Alkansulfonyloxy-imidazo[4,5-c]pyridazine, Alkansulfonyloxy-imidazo[4,5-d]pyridazine, Alkansulfonyloxy-imidazo[4,5-b]pyrazine, Alkoxy-imidazo[4,5-b]pyridine, Alkoxy-imidazo[4,5-c]pyridine, Alkoxypurine, Alkoxy-imidazo[4,5-c]pyridazine, Alkoxy-imidazo-[4,5-b]pyrazine, Chlor-purine, Chlor-imidazo[4,5-c]pyridazine, Chlor-imidazo[4,5-d]pyridazine oder Chlor-imidazo-[4,5-b]pyrazine der oben allgemeinen Formel I, deren Tautomeren, deren Säureadditionssalze, insbesondere deren physiologisch verträgliche Säureadditionssalze mit anorganischen oder organischen Säuren, und Verfahren zu ihrer Herstellung und diese Verbindungen enthaltende Arzneimittel.

Für die bei der Definition der Reste eingangs erwähnten Bedeutungen kommt beispielsweise

für $R_1$ die Bedeutung der Cyanomethoxy-, Hydroxycarbonylmethoxy-, Methoxycarbonylmethoxy-, Ethoxycarbonylmethoxy-, Propoxycarbonylmethoxy-, Isopropoxycarbonylmethoxy-, Methansulfonyloxy-, Ethansulfonyloxy-, Propansulfonyloxy-, Isopropansulfonyloxy-, n-Butansulfonyloxy-, Methansulfonylamino-, Ethansulfonylamino-, n-Propansulfonylamino-, N-Methylmethansulfonyl-amino-, N-Isopropyl-methansulfonylamino-, N-Ethylethansulfonylamino-, N-n-Propyl-ethansulfonylamino-, N-Methyl-propan-sulfonylamino-, N-Ethyl-propansulfonylamino-, Vinyloxy-, Allyloxy-, Crotyloxy-, Penten-(2)-yloxy-, Penten-(3)-yloxy- oder Propargyloxygruppe,

für $R_2$ die der Methoxy-, Ethoxy-, Propoxy-, Isopropoxy-, Dimethylamino-, Diethylamino-, Dipropylamino-, Diisopropylamino-, Ethyl-methylamino-, Ethyl-propylamino-, Vinyloxy-, Allyloxy-, Crotyloxy-, Penten-(2)-yloxy-, Penten-(3)-yloxy- oder Propargyloxygruppe und

für $R_3$ die der Methyl-, Ethyl-, n-Propyl-, Isopropyl- oder n-Butylgruppe in Betracht.

Bevorzugte Verbindungen der obigen allgemeinen Formel I sind diejenigen, in denen A bis D, $R_1$ und $R_2$ wie eingangs definiert sind und $R_3$ eine Methylgruppe, $R_4$ und $R_5$ eine Allyl- oder Propargylgruppe darstellen, insbesondere diejenigen Verbindungen der allgemeinen Formel I, in der $R_1$ in 4-Stellung und $R_2$ in 2-Stellung des Phenylkerns stehen, und deren Säureadditionssalze, insbesondere deren physiologisch verträgliche Säureadditionssalze.

Besonders bevorzugte Verbindungen der obigen allgemeinen Formel I sind jedoch die neuen Imidazo[4,5-b]pyridin-5-one, Imidazo[4,5-c]pyridin-4-one, Imidazo[4,5-c]pyridin-6-one, Imidazo[4,5-c]pyridazin-6-one, Imidazo[4,5-d]pyridazin-4-one, Imidazo[4,5-b]pyrazin-5-one, Purin-2-one, Purin-6-one, Purin-2,6-dione und Imidazo[4,5-e]triazin-6-one, in denen $R_1$ eine Propargyloxy-, Methansulfonyloxy-, Methansulfonylamino- oder N-Methyl-methansulfonylaminogruppe oder, wenn A, B, C und D zusammen mit dem ankondensierten Imidazolring kein Imidazo[4,5-c]pyridin bilden, auch eine Allyloxygruppe und

$R_2$ eine Methoxy- oder Dimethylaminogruppe bedeuten, insbesondere diejenigen Verbindungen der allgemeinen Formel I, in der $R_1$ in 4-Stellung und $R_2$ in 2-Stellung des Phenylkerns stehen, und deren Säureadditionssalze, insbesondere deren physiologisch verträgliche Säureadditionssalze.

Erfindungsgemäß erhält man die neuen Verbindungen nach folgenden Verfahren:

a) Cyclisierung einer gegebenenfalls im Reaktionsgemisch hergestellten Verbindung der allgemeinen Formel

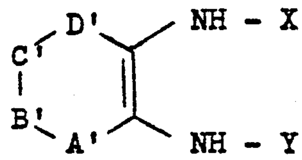

,(II)

in der

A' bis D' die für A bis D eingangs erwähnten Bedeutungen besitzen oder einer der Reste A' bis D' eine durch eine Schutzgruppe geschützte Oxymethingruppe darstellt, einer der Reste X oder Y ein Wasserstoffatom und der andere der beiden Reste X und Y oder beide Reste X und Y eine Gruppe der Formel

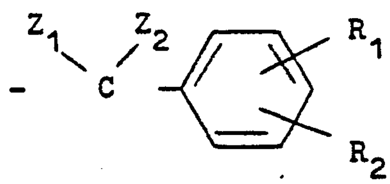

darstellen, in der

$R_2$ wie eingangs definiert ist,

$R_1$' die für $R_1$ eingangs erwähnten Bedeutungen besitzt oder eine durch eine Schutzgruppe geschützte Hydroxygruppe darstellt,

$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, und gegebenenfalls anschließende Abspaltung einer verwendeten Schutzgruppe.

Als Schutzgruppen kommen übliche Schutzgruppen für eine Hydroxygruppe in Betracht, z.B. eine Acylgruppe wie die Acetyl-, Propionyl- oder Benzoylgruppe oder eine Benzylgruppe.

Die Cyclisierung wird zweckmäßigerweise in einem Lösungsmittel oder Lösungsmittelgemisch wie Wasser, Ethanol, Isopropanol, Eisessig, Benzol, Chlorbenzol, Toluol, Xylol, Glycol, Glycol-monomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester, Amid oder Methojodid, beipielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Salzsäure, Phosphorsäure, Polyphosphorsäure, Essigsäureanhydrid oder gegebenenfalls auch in Gegenwart einer Base wie Natriumhydroxid, Kaliumäthylat oder Kalium-tert.butylat durchgeführt. Die Cyclisierung kann jedoch auch ohne Lösungsmittel und/ oder Kondensationsmittel durchgeführt werden.

Eine bei der Umsetzung gegebenenfalls verwendete Schutzgruppe wird anschließend hydrolytisch oder hydrogenolytisch abgespalten. Eine verwendete Schutzgruppe kann aber auch, sofern als Schutzgruppe eine Acylgruppe verwendet wird, während der Umsetzung abgespalten werden.

Die hydrolytische Abspaltung einer verwendeten Schutzgruppe wird zweckmäßigerweise in einem wässrigen oder alkoholischen Lösungsmittel wie Wasser, Wasser/Methanol, Wasser/Dioxan oder Methanol in Gegenwart einer Säure wie Salzsäure oder einer Base wie Natronlauge oder Ammoniak bei Temperaturen bis zur Siedetemperatur des verwendeten Lösungsmittels durchgeführt.

Die hydrogenolytische Abspaltung eines verwendeten Schutzrestes wird in einem Lösungsmittel wie Essigester, Eisessig oder Methanol mit Wasserstoff in Gegenwart eines Hydrierungskatalysators wie Platin oder Palladium/Kohle gegebenenfalls unter Zusatz einer Base wie Natron- oder Kalilauge durchgeführt.

b) Umlagerung eines N-Oxids der allgemeinen Formel

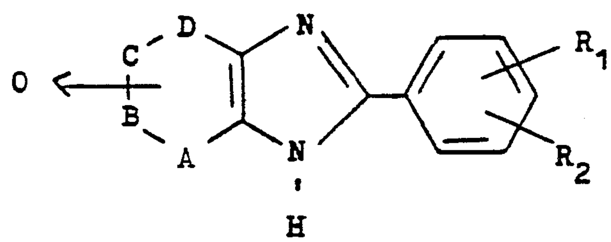

,(III)

in der
A, B, C, D, $R_1$ und $R_2$ wie eingangs definiert sind, und gegebenenfalls anschließende Hydrolyse.

Die Umsetzung wird gegebenenfalls in einem Lösungmittel wie Benzol in Gegenwart eines Acylierungsmittels wie Essigsäureanhydrid oder Propionsäureanhydrid, wobei dieses zweckmäßigerweise gleichzeitig auch als Lösungsmittel verwendet wird, bei erhöhten Temperaturen, vorzugsweise jedoch bei der Siedetemperatur des verwendeten Lösungsmittels, durchgeführt wird.

Die gegebenenfalls anschließende Hydrolyse wird zweckmäßigerweise in Wasser, Wasser/Methanol Wasser/Dioxan oder Methanol in Gegenwart einer Säure wie Salzsäure oder einer Base wie Natronlauge oder Ammoniak bei der Siedetemperatur des Reaktionsgemisches durchgeführt.

c) Zur Herstellung von Verbindungen der allgemeinen Formel I, in der $R_1$ eine Alkansulfonyloxy-, Alkansulfonylamino- oder N-Alkyl-alkansulfonylaminogruppe und/oder eine der Gruppen A, B, C oder D eine $R_3SO_2$-O-C-Gruppe darstellen:

Umsetzung einer Verbindung der allgemeinen Formel

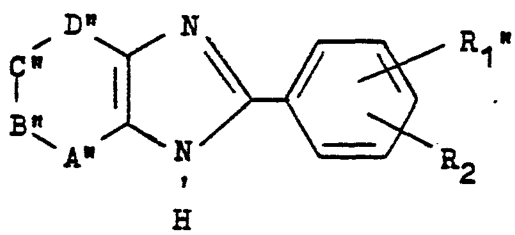

,(IV)

in der
$R_2$ wie eingangs definiert ist,
A", B", C", D" und $R_1$" die für A, B, C, D und $R_1$ eingangs erwähnten Bedeutungen besitzen, wobei jedoch entweder $R_1$" eine Hydroxy-, Amino- oder Alkylaminogruppe oder einer der Reste A", B", C" oder D" eine HO-C-Gruppe darstellen muß, mit einem Sulfonylhalogenid der allgemeinen Formel

$$Hal - SO_2 - R_3 \quad ,(V)$$

in der
$R_3$ wie eingangs definiert ist und
Hal ein Chlor- oder Bromatom darstellt, und gegebenenfalls anschließende partielle Hydrolyse.

Die Umsetzung wird in einem Lösungsmittel wie Wasser, Tetrahydrofuran, Dioxan oder Dimethylformamid in Gegenwart eines säurebindenden Mittels wie Natriumhydroxid, Natriumhydrid, Kalium-tert.butylat, Triäthylamin, Ethyl-di-isopropylamin

oder Pyridin, wobei die drei letzteren auch als Lösungsmittel dienen können, bei Temperaturen zwischen 0 und 100°C, vorzugsweise bei Temperaturen zwischen 20 und 50°C, durchgeführt.

Zur Herstellung einer Verbindung der allgemeinen Formel I, in der $R_1$ eine Alkansulfonyloxygruppe darstellt, ist es von Vorteil, über die partielle Hydrolyse eines so erhaltenen Reaktionsproduktes zu gehen.

Die gegebenenfalls anschließende Hydrolyse wird zweckmäßigerweise in Wasser, Wasser/Methanol, Wasser/Dioxan oder in einem Alkohol wie Methanol, Ethanol oder Isopropanol in Gegenwart einer Säure wie Salzsäure durchgeführt, wobei gegebenenfalls bis zur Siedetemperatur des jeweiligen Lösungsmittels erhitzt wird.

d) Zur Herstellung von Verbindungen der allgemeinen Formel I, in der $R_1$ oder $R_1$ und $R_2$ eine Alkenyloxy- oder Alkinyloxygruppe oder $R_2$ eine Alkoxygruppe darstellen:

Umsetzung einer Verbindung der allgemeinen Formel

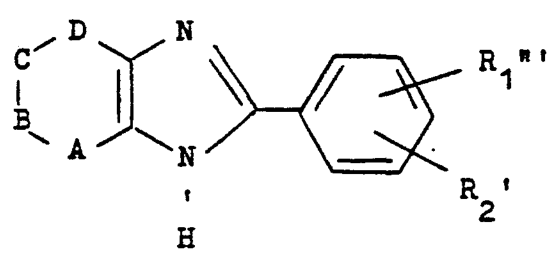

,(VI)

in der

A, B, C und D wie eingangs definiert sind,

$R_1'''$ und $R_2'$ die für $R_1$ und $R_2$ eingangs erwähnten Bedeutungen besitzen, wobei jedoch mindestens einer der Reste $R_1'''$ oder $R_2'$ eine Hydroxygruppe darstellen muß, mit einem Halogenid der allgemeinen Formel

$$Hal - R_6 \quad ,(VII)$$

in der
$R_6$ die für $R_3$, $R_4$ und $R_5$ eingangs erwähnten Bedeutungen besitzt und
Hal ein Chlor-, Brom- oder Jodatom darstellt.

Die Umsetzung wird zweckmäßigerweise in einem Lösungsmittel wie Tetrahydrofuran, Dioxan, Dimethylformamid, Sulfolan, Dimethylsulfoxid oder Ethylenglycoldimethylether vorzugsweise in Gegenwart eines säurebindenden Mittels wie Kaliumkarbonat, Kalium-tert.butylat oder Natriumhydrid bei Temperaturen zwischen 0 und 100°C, vorzugsweise jedoch bei Temperaturen zwischen 20 und 50°C, durchgeführt.

Die erhaltenen Verbindungen der allgemeinen Formel I können anschließend gewünschtenfalls in ihre physiologisch verträglichen Säureadditionssalze mit anorganischen oder organischen Säuren übergeführt werden. Als Säuren kommen hierfür beispielsweise Salzsäure, Bromwasserstoffsäure, Schwefelsäure, Phosphorsäure, Furmarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formeln II bis VII sind teilweise literaturbekannt bzw. erhält man sie nach literaturbekannten Verfahren. So erhält man beispielsweise die als Ausgangsstoffe verwendeten Verbindungen der allgemeinen Formel II durch Acylierung der entsprechenden o-Diaminoverbindungen bzw. durch Reduktion der entsprechenden Acylamino-nitro-Verbindungen, die Verbindungen der allgemeinen Formel IV oder VI durch anschließenden Ringschluß und die Verbindungen der allgemeinen Formel III durch zusätzliche N-Oxidation (siehe BE-PS 810.545 und EP-A-0.024.290).

Wie bereits eingangs erwähnt weisen die neuen Verbindungen der allgemeinen Formel I, deren Tautomere und deren physiologisch verträgliche Säureadditionssalze bei einer langen Wirkungsdauer überlegene pharmakologische Eigenschaften auf, insbesondere eine blutdrucksenkende und/oder positiv inotrope Wirkung.

Beispielweise wurden die Verbindungen

A = 8-(2-Methoxy-4-methansulfonyloxy-phenyl)-3(1)H-purin-2-on,
B = 2-(2-Methoxy-4-methansulfonyloxy-phenyl)-5-methansulfonyloxy-imidazol[4,5-b]pyridin,
C = 2-(2-Methoxy-4-methansulfonyloxy-phenyl)-4H-imidazo-[4,5-b]pyridin-5-on,
D = 2-(2-Methoxy-4-methansulfonyloxy-phenyl)-5H-imidazo-[4,5-c]pyridin-4-on-hydrochlorid,
E = 2-(2-Methoxy-4-methansulfonyloxy-phenyl)-4-methansulfonyloxy-imidazo[4,5-c]pyridin,
F = 2-(2-Methoxy-4-propargyloxy-phenyl)-5H-imidazo[4,5-c]-pyridin-4-on und
G = 2-(2-Methoxy-4-methansulfonyloxy-phenyl)-4H-imidazo-[4,5-b]pyrazin-5-on

auf ihre biologischen Eigenschaften wie folgt untersucht:

1.) <u>Bestimmung der Blutdruckwirkung und der positiv inotropen Wirkung an der narkotisierten Katze</u>

Die Untersuchungen wurden an Katzen durchgeführt, die mit Pentobarbital-Natrium (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde

der Kontraktilitätsparamter $dp/dt_{max}$ mittels eines Analogdifferenzierers gewonnen. Die zu untersuchenden Substanzen wurden in eine Vena femoralis injiziert. Als Lösungsmittel diente physiologische Kochsalz-Lösung oder Polydiol 200. Jede Substanz wurde an mindestens 3 Katzen geprüft, Dosis 2 mg/kg i.v..
Die nachfolgende Tabelle enthält die Mittelwerte:

| Substanz | Zunahme von $dp/dt_{max}$ in % | Blutdrucksenkung in mm Hg | Wirkungsdauer (Halbwertszeit) in Minuten |
|---|---|---|---|
| A | + 48 | - 8/- 6 | 42 |
| B | + 53 | - 50/-42 | 37 |
| C | + 158 | - 54/-54 | 31 |
| D | + 117 | - 42/-42 | 31 |
| E | + 87 | - 35/-27 | 29 |
| F | + 138 | - 59/-47 | 8 |
| G | + 100 | - 26/-27 | 5 |

Die neuen Verbindungen sind gut verträglich, so konnte bei der Untersuchung der Substanzen A bis G keinerlei herztoxische Wirkungen bzw. Kreislaufschäden beobachtet werden.

Aufgrund ihrer pharmakologischen Eigenschaften eignen sich die erfindungsgemäß hergestellten Verbindungen der allgemeinen Formel I sowie deren physiologisch verträgliche Säureadditionssalze zur Behandlung von Herzinsuffizienzen unterschiedlicher Genese, da sie die Kontraktionskraft des Herzens steigern bzw. durch zusätzliche Blutdrucksenkung die Entleerung des Herzens erleichtern.

Hierzu lassen sich die neuen Verbindungen sowie deren physiologisch verträgliche Säureadditionssalze, gegebenenfalls in Kombination mit anderen Wirksubstanzen, in die üblichen pharmazeutischen Anwendungsformen wie Tabletten, Dragées, Pulver, Suppositorien, Suspensionen, Ampullen oder Tropfen einarbeiten. Die Einzeldosis beträgt hierbei 1-4 x täglich 0,1 - 5 mg/kg Körpergewicht, vorzugsweise jedoch 0,5 - 2,0 mg/kg Körpergewicht.

Die nachfolgenden Beispiele sollen die Erfindung näher erläutern:

Beispiel 1

2-(2-Methoxy-4-methansulfonyloxy-phenyl)-4H-imidazo[4,5-b]-pyridin-5-on

a) 2-(2-Methoxy-4-methansulfonyloxy-phenyl)-imidazo[4,5-b]-pyridin-4-oxid

3,19 g 2-(2-Methoxy-4-methansulfonyloxy-phenyl)-imidazo-[4,5-b]pyridin werden in 25 ml Trifluoressigsäure gelöst und dazu 5 ml 30%iges Wasserstoffperoxid getropft. Anschließend erhitzt man eine Stunde zum Rückfluß und dampft dann auf etwa 1/10 des Volumens ein. Der Rückstand wird auf Eis gegossen und digeriert bis er durchkristallisiert ist.
Ausbeute: 2,1 g (63 % der Theorie),
Schmelzpunkt: 110-125°C.

b) 2-(2-Methoxy-4-methansulfonyloxy-phenyl)-4H-imidazo-[4,5-b]pyridin-5-on

2 g des oben erhaltenen Produktes werden in 20 ml Acetanhydrid gelöst und 2,5 Stunden zum Rückfluß erhitzt. Die Lösung wird im Vakuum eingedampft, der Rückstand mit 10 ml 2n Salzsäure versetzt und unter Rühren 20 Minuten zum Sieden erhitzt. Nach dem Abkühlen wird das Produkt durch Zusatz von Natriumacetat gefällt und durch Chromatographie an Kieselgel (Elutionsmittel: Methylenchlorid/Ethanol 1:0 bis 1:0,1) gereinigt.
Ausbeute: 0,72 g (36 % der Theorie),
Schmelzpunkt: 258-259°C (Zersetzung).

Analog werden folgende Verbindungen erhalten:

2-(2-Methoxy-4-propansulfonyloxy-phenyl)-4H-imidazo[4,5-b]-pyridin-5-on
Schmelzpunkt: 193-195°C

2-(2-Methoxy-4-ethansulfonyloxy-phenyl)-4H-imidazo[4,5-b]-pyridin-5-on
Schmelzpunkt: 241-244°C

2-(2-Methoxy-4-isopropylsulfonyloxy-phenyl)-4H-imidazo[4,5-b]-pyridin-5-on
Schmelzpunkt: 258-261°C

2-(2-Methoxy-4-n-butylsulfonyloxy-phenyl)-4H-imidazo[4,5-b]-pyridin-5-on
Schmelzpunkt: 203-205°C

## Beispiel 2

2-(2-Methoxy-4-methansulfonylamino-phenyl)-4H-imidazo[4,5-b]-pyridin-5-on

2,7 g 5-Amino-6-(2-methoxy-4-methansulfonylamino-benzoyl-amino)-2-pyridon werden in 50 ml Eisessig 1,5 Stunden zum Rückfluß erhitzt. Danach wird im Vakuum eingedampft und der Rückstand über Kieselgel (Elutionsmittel: Methylenchlorid/-Ethanol 1:0 bis 1:0,1) gereinigt.
Ausbeute: 0,1 g (4 % der Theorie),
Schmelzpunkt: über 255°C.

| | | | | | | | | |
|---|---|---|---|---|---|---|---|---|
| Ber.: | C | 50,29 | H | 4,22 | N | 16,76 | S | 9,54 |
| Gef.: | | 49,95 | | 4,48 | | 16,52 | | 9,49 |

Analog werden folgende Verbindungen erhalten:

2-(2-Methoxy-4-methansulfonylamino-phenyl)-4H-imidazo[4,5-d]-pyridazin-4-on
Schmelzpunkt: 310-314°C (Zers.)

8-(2-Methoxy-4-methansulfonyloxy-phenyl)-1H,3H-purin-2,6-dion
Schmelzpunkt: 305-307°C (Zers.)

8-(2-Methoxy-4-methansulfonylamino-phenyl)-3(1)H-purin-2-on
Schmelzpunkt: 230-232°C

8-(2-Methoxy-4-cyanmethyloxy-phenyl)-3(1)H-purin-2-on

8-(2-Methoxy-4-propargyloxy-phenyl)-3(1)H-purin-2-on
Schmelzpunkt: > 300°C

| | | | | | | |
|---|---|---|---|---|---|---|
| Ber.: | C | 60,80 | H | 4,08 | N | 18,91 |
| Gef.: | | 60,96 | | 4,09 | | 18,83 |

2-(2-Methoxy-4-cyanmethyloxy-phenyl)-5H-imidazo[4,5-c]pyridin-4-on

2-(2-Methoxy-4-ethoxycarbonylmethyloxy-phenyl)-5H-imidazo-[4,5-c]pyridin-4-on

2-(2-Methoxy-4-carboxymethyloxy-phenyl)-5H-imidazo[4,5-c]-pyridin-4-on.

2-(2-Methoxy-4-allyloxy-phenyl)-5H-imidazo[4,5-c]pyridin-4-on

8-[2-Dimethylamino-4-(N-methyl-N-methansulfonylamino)-phenyl]-3(1)H-purin-2-on

2-(2-Dimethylamino-4-methansulfonylamino-phenyl)-4H-imidazo-[4,5-b]pyridin-5-on

<u>Beispiel 3</u>

<u>8-(2-Methoxy-4-methansulfonyloxy-phenyl)-3(1)H-purin-2-on</u>

1 g 8-(2-Methoxy-4-hydroxy-phenyl)-3(1)H-purin-2-on wird in 10 ml Pyridin suspendiert. Unter Rühren werden 2 ml Methansulfonsäurechlorid zugetropft. Es wird noch 15 Minuten nachgerührt, dann mit 3 ml Wasser versetzt und im Vakuum eingedampft. Der Rückstand wird mit Wasser versetzt und das ausgefallene Festprodukt über Kieselgel (Elutionsmittel: Methanol) gereinigt.
Ausbeute: 0,11 g (8,5 % der Theorie),
Schmelzpunkt: 233-235°C (Zersetzung).

Analog werden folgende Verbindungen erhalten:

2-(2-Methoxy-4-methansulfonyloxy-phenyl)-4H-imidazo[4,5-d]-pyridazin-4-on (Bei der Aufarbeitung wird mit ethanolischer Salzsäure versetzt und digeriert)
Schmelzpunkt: 294-296°C (Zers.)

2-(2-Methoxy-4-methansulfonyloxy-phenyl)-5(7)H-imidazo-[4,5-e]triazin-6-on

2-(2-Methoxy-4-methansulfonyloxy-phenyl)-5H-imidazo[4,5-c]-pyridin-6-on (Bei der Aufarbeitung wird mit Salzsäure versetzt)

8-(2-Dimethylamino-4-methansulfonyloxy-phenyl)-3(1)H-purin-2-on

2-(2-Dimethylamino-4-methansulfonyloxy-phenyl)-5H-imidazo-[4,5-c]pyridin-4-on

8-(2-Dimethylamino-4-methansulfonylamino-phenyl)-1H,3H-purin-2,6-dion

6-Methoxy-2-(2-methoxy-4-methansulfonyloxy-phenyl)-imidazo-[4,5-c]pyridin
Schmelzpunkt: 231°C (Zers.)

2-(2-Methoxy-4-methansulfonylamino-phenyl)-4H-imidazo[4,5-d]-pyridazin-4-on
Schmelzpunkt: 310-314°C (Zers.)

2-(2-Methoxy-4-methansulfonyloxy-phenyl)-5H-imidazo[4,5-c]-pyridazin-6-on

2-(2-Methoxy-4-methansulfonylamino-phenyl)-5H-imidazo[4,5-c]-pyridazin-6-on
Schmelzpunkt: 320-322°C (Zers.)

2-Methoxy-8-(2-ethoxy-4-methansulfonyloxy-phenyl)-purin
Schmelzpunkt: 127-130°C

2-Methoxy-8-(2-propoxy-4-methansulfonyloxy-phenyl)-purin
Schmelzpunkt: 173-175°C

8-(2-Ethoxy-4-methansulfonyloxy-phenyl)-3(1H)-purin-2-on

8-(2-Propoxy-4-methansulfonyloxy-phenyl)-3(1H)-purin-2-on

## Beispiel 4

2-(2-Methoxy-4-methansulfonyloxy-phenyl)-4-methansulfonyloxy-imidazo[4,5-c]pyridin

1 g 2-(2-Methoxy-4-hydroxy-phenyl)-5H-imidazo[4,5-c]pyridin-4-on werden in 10 ml Pyridin suspendiert. Nach Zugabe von 2 ml Methansulfonsäurechlorid wird weitere 2 Stunden bei Raumtemperatur gerührt. Das Reaktionsgemisch wird mit Wasser versetzt, hierbei fällt das Produkt aus.
Ausbeute: 1,08 g (77 % der Theorie),
Schmelzpunkt: Zersetzung ab 164°C.

| | | | | | | | | |
|---|---|---|---|---|---|---|---|---|
| Ber.: | C | 43,58 | H | 3,66 | N | 10,16 | S | 15,51 |
| Gef.: | C | 43,30 | | 3,75 | | 10,05 | | 15,73 |

Analog wird folgende Verbindung erhalten:

2-(2-Methoxy-4-methansulfonyloxy-phenyl)-6-methansulfonyloxy-imidazo[4,5-b]pyridin
Schmelzpunkt: 188-189°C

Beispiel 5

2-(2-Methoxy-4-methansulfonyloxy-phenyl)-5H-imidazo[4,5-c]-pyridin-4-on-hydrochlorid

0,65 g 2-(2-Methoxy-4-methansulfonyloxy-phenyl)-4-methansulfonyloxy-imidazo[4,5-c]pyridin werden in 35 ml ethanolischer Salzsäure gelöst und dann noch eine Stunde bei Raumtemperatur gerührt. Der ausgefallene Niederschlag wird abgesaugt und mit Ethanol gewaschen.
Ausbeute: 0,53 g (91 % der Theorie),
Schmelzpunkt: 193-195°C (Zersetzung).

Beispiel 6

2-(2-Methoxy-4-propargyloxy-phenyl)-5H-imidazo[4,5-c]pyridin-4-on

1 g 2-(2-Methoxy-4-hydroxy-phenyl)-5H-imidazo[4,5-c]pyridin-4-on werden in 30 ml Sulfolan suspendiert, 1,41 g Kaliumcarbonat zugefügt und bei Raumtemperatur gerührt. Nach 15 Minuten und nach einer Stunde werden jeweils 0,9 ml Propargylbromid zugegeben, und dann noch 1,25 Stunden weiter gerührt. Das Lösungsmittel wird im Vakuum weitgehend abgedampft und der Rückstand mit Methylenchlorid verrührt. Nach Abfiltrieren des Niederschlages und Waschen mit Wasser wird das in der organischen Phase enthaltene Produkt über eine Kieselgelsäule (Elutionsmittel: Methylenchlorid/Ethanol 1:0 bis 1:0,03) gereinigt. Das Produkt kristallisiert mit einem halben Mol Ethanol.
Ausbeute: 0,07 g (7 % der Theorie),
Schmelzpunkt: 178-180°C.

Beispiel 7

2-(2-Methoxy-4-methansulfonyloxy-phenyl)-4H-imidazo[4,5-b]-pyrazin-5-on

Hergestellt analog Beispiel 1b aus 0,75 g 2-(2-Methoxy-4-methansulfonyloxy-phenyl)-imidazo[4,5-b]pyrazin-4-oxid (1,5 Stunden mit Acetanhydrid erhitzt).
Ausbeute: 0,13 g (17 % der Theorie),
Schmelzpunkt: über 260°C.

| | | | | | | | | |
|---|---|---|---|---|---|---|---|---|
| Ber.: | C | 46,44 % | H | 3,60 | N | 16,66 | S | 9,55 |
| Gef.: | | 46,34 | | 3,86 | | 16,50 | | 9,29 |

Beispiel 8

2-(2-Methoxy-4-methansulfonyloxy-phenyl)-5-methansulfonyloxy-imidazol[4,5-b]pyridin und

2-(2-Methoxy-4-hydroxy-phenyl)-5-methansulfonyloxy-imidazo-[4,5-b]pyridin

1,6 g 2-(2-Methoxy-4-hydroxy-phenyl)-4H-imidazo[4,5-b]pyridin-5-on werden in 25 ml Pyridin gelöst, mit 0,6 ml Methansulfonsäurechlorid versetzt und über Nacht bei Raumtemperatur gerührt. Man versetzt mit 50 ml Eis und stellt schließlich mit 20 ml konzentrierter Salzsäure sauer. Man erhitzt 1,5 Stunden auf dem Dampfbad, kühlt ab und reinigt das ausgefallene Produkt durch Chromatographie an Kieselgel (Elutionsmittel: Methylenchlorid/Ethanol 1:0,01 bis 1:0,2)
Fraktion I:
Ausbeute: 0,18 g (10 % der Theorie) 2-(2-Methoxy-4-methansulfonyloxy-phenyl)-5-methansulfonyloxy-imidazol[4,5-b]pyridin
Schmelzpunkt: 223-225°C.

Fraktion II:
Ausbeute: 0,21 g (15 % der Theorie) 2-(2-Methoxy-4-hydroxy-phenyl)-5-methansulfonyloxy-imidazo[4,5-b]pyridin
Schmelzpunkt: 227-228°C.

Beispiel 9

2-(2-Methoxy-4-hydroxy-phenyl)-4H-imidazo[4,5-b]pyridin-5-on

3,95 g 5-Nitro-6-(2-methoxy-4-benzyloxy-benzoylamino)-2-pyridon werden in 350 ml Eisessig nach Zusatz von 1 g 10%iger Palladiumkohle 1,7 Stunden bei 80°C und 5 bar Druck hydriert. Anschließend wird das Reaktionsgemisch 1,3 Stunden bei 100°C gerührt. Man filtriert vom Katalysator ab und läßt abkühlen. Das ausgefallene Produkt wird mit Ether gewaschen. Durch Eindampfen des Filtrats und Versetzen mit Ether erhält man weitere Fraktionen.
Ausbeute: 2,3 g (60 % der Theorie),
Schmelzpunkt: über 270°C.

| | | | | | | |
|---|---|---|---|---|---|---|
| Ber.: | C | 54,10 | N | 5,07 | N | 11,13 |
| Gef.: | | 54,38 | | 4,96 | | 11,04 |

Beispiel 10

2-(2-Methoxy-4-methansulfonylamino-phenyl)-6-hydroxy-imidazo[4,5-b]pyridin

0,5 g 2,3-Diamino-5-acetoxy-pyridin und 0,83 g 2-Methoxy-4-methansulfonylamino-benzoesäure werden 1 Stunde in 20 ml Phosphoroxychlorid zum Rückfluß erhitzt. Das Reaktionsgemisch wird mit Eiswasser zersetzt und anschließend mit konzentriertem wäßrigen Ammoniak auf pH 7 gebracht. Das Produkt wird dann mit Essigester extrahiert und säulenchromatographisch gereinigt (Kieselgel, Elutionsmittel: Methylenchlorid/Ethanol = 1:0,02 bis 1:0,1)
Ausbeute: 0,06 g (6 % der Theorie),
Schmelzpunkt: 225°C (Zers.).

Analog wird folgende Verbindung erhalten:

2-Methoxy-8-(2-methoxy-4-propargyloxy-phenyl)-purin
Schmelzpunkt: 148-150°C

Beispiel 11

2-Chlor-8-(2-methoxy-4-propargyloxy-phenyl)-purin

2,5 g 2-Chlor-8-(2-methoxy-4-propargyloxy-benzoylamino)-5-amino-pyrimidin werden in einer Mischung aus 50 ml 2 n Natronlauge und 50 ml Ethanol gelöst und 2 Stunden am Rückfluß gekocht. Man verdünnt mit 50 ml Eiswasser und erhält durch Ansäuern mit Eisessig einen Niederschlag. Es wird abgesaugt, in Aceton/Methylenchlorid aufgenommen, über Magnesiumsulfat getrocknet und im Vakuum zur Trockne eingedampft. Der Rückstand wird mit Ether verrieben und abgesaugt.
Ausbeute: 1,4 g (64 % der Theorie),
Schmelzpunkt: 229-231°C (Zers.)

Beispiel 12

8-(2-Methoxy-4-methansulfonyloxy-phenyl)-purin-6-on

a) 6-Methansulfonyloxy-8-(2-methoxy-4-methansulfonyloxy-phenyl)-purin

Hergestellt analog Beispiel 4. Das Produkt wird ohne weitere Reinigung weiterverarbeitet.

b) 8-(2-Methoxy-4-methansulfonyloxy-phenyl)-purin-6-on

Hergestellt analog Beispiel 5 aus 6-Methansulfonyloxy-8-(2-methoxy-4-methansulfonyloxy-phenyl)-purin.
Schmelzpunkt: 288-291°C

Beispiel 13

8-(2-Methoxy-4-methansulfonyloxy-phenyl)-1H,3H-purin-2,6-dion

Hergestellt analog Beispiel 3 in wäßriger Natronlauge, wobei das pH zwischen 10 und 10,5 gehalten wird.
Schmelzpunkt: 305-307°C (Zers.).

Analog werden folgende Verbindungen erhalten:

1,3-Dimethyl-8-(2-methoxy-4-methansulfonyloxy-phenyl)-1H,3H-purin-2,6-dion
Schmelzpunkt: 239-241°C

1,3-Dimethyl-8-(2-dimethylamino-4-methansulfonylamino-phenyl)-1H,3H-purin-2,6-dion

8-(2-Dimethylamino-4-methansulfonylamino-phenyl)-1H,3H-purin-2,6-dion

Beispiel 14

8-(2-Methoxy-4-hydroxy-phenyl)-purin-2-on

3,5 g 2-Benzyloxy-8-(2-methoxy-4-benzyloxy-phenyl)-purin (hergestellt gemäß Verfahren a)) werden in 100 ml Ethanol in Gegenwart von 1 g 20%iger Palladiumkohle bei 50°C und 5 bar Druck hydriert. Nach Abfiltrieren des Katalysators wird das Filtrat eingedampft, der Rückstand mit 2n Natronlauge extrahiert und das Produkt durch Ansäuern mit Eisessig gefällt. Die weitere Reinigung erfolgt durch Verreiben mit Aceton.
Ausbeute: 0,22 g (10,6 % der Theorie),
Schmelzpunkt: 250°C (Zers.).

Analog wird folgende Verbindung erhalten:

1,3-Dimethyl-8-(2-methoxy-4-hydroxy-phenyl)-1H,3H-purin-2,6-dion

(Hydrogenolyse in Gegenwart von zwei Äquivalenten Kaliumhydroxid)

Schmelzpunkt: über 310°C

| | | | | | | | |
|---|---|---|---|---|---|---|---|
| Ber.: | C | 56,00 | % | H | 4,66 | N | 18,50 |
| Gef.: | | 55,63 | | | 4,67 | | 18,53 |

Beispiel A

Tabletten zu 100 mg 2-(2-Methoxy-4-methansulfonyloxy-phenyl)-5H-imidazo[4,5-c]pyridin-4-on-hydrochlorid

Zusammensetzung

1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Feuchtsiebung: 1,5 mm

Trocknen: Umlufttrockenschrank 50°C

Trockensiebung: 1 mm

Dem Granulat die restlichen Hilfsstoffe zumischen und Endmischung zu Tabletten verpressen.

Tablettengewicht: 175 mg

Stempel: 8 mm

Beispiel B

Dragées zu 50 mg 2-(2-Methoxy-4-methansulfonyloxy-phenyl)-5H-imidazo[4,5-c]pyridin-4-on-hydrochlorid

Zusammensetzung:

1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

| | |
|---|---|
| Feuchtsiebung: | 1,0 mm |
| Trockensiebung: | 1,0 mm, |
| Trocknung: | 50°C im Umlufttrockenschrank |

Granulat und restliche Hilfsstoffe mischen und zu Kernen verpressen.

| | |
|---|---|
| Kerngewicht: | 80 mg |
| Stempel: | 6 mm |
| Wölbungsradius: | 5 mm |

Die fertigen Kerne werden auf übliche Weise mit einem Zuckerüberzug im Dragierkessel versehen.

Drageegewicht: 120 mg

Beispiel C

Suppositorien zu 75 mg 2-(2-Methoxy-4-methansulfonyloxy-phenyl)-5H-imidazo[4,5-c]pyridin-4-on-hydrochlorid

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

Herstellungsverfahren:

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

Beispiel D

Ampullen zu 50 mg 2-(2-Methoxy-4-methansulfonyloxy-phenyl)-5H-imidazo[4,5-c]pyridin-4-on-hydrochlorid

1 Ampulle enthält:

| | |
|---|---|
| Wirksubstanz | 50,0 mg |
| Sorbit | 250,0 mg |
| Dest. Wasser ad | 5,0 ml |

Herstellungsverfahren:

Die Wirksubstanz und Sorbit werden in dest. Wasser gelöst, dann wird das angegebene Volumen aufgefüllt und steril filtriert.

Abfüllung: in Ampullen zu 5 ml

Sterilisation: 20 Minuten bei 120°C

Beispiel E

Tropfen zu 250 mg 2-(2-Methoxy-4-methansulfonyloxy-phenyl)-5H-imidazo[4,5-c]pyridin-4-on-hydrochlorid

| | | | |
|---|---|---|---|
| Wirksubstanz | | 5,0 | g |
| p-Oxybenzoesäuremethylester | | 0,035 | g |
| p-Oxybenzoesäurepropylester | | 0,015 | g |
| Anisöl | | 0,05 | g |
| Menthol | | 0,06 | g |
| Saccharin-Natrium | | 1,0 | g |
| Glycerin | | 10,0 | g |
| Ethanol | | 40,0 | g |
| Dest. Wasser | ad | 100,0 | ml |

Herstellungsverfahren:

Die Benzoesäureester werden in Ethanol gelöst und anschließend das Anisöl und das Menthol zugegeben. Dann wird die Wirksubstanz, Glycerin und Saccharin-Natrium im Wasser gelöst zugegeben. Die Lösung wird anschließend klar filtriert.

## Patentansprüche

1. Imidazole der allgemeinen Formel

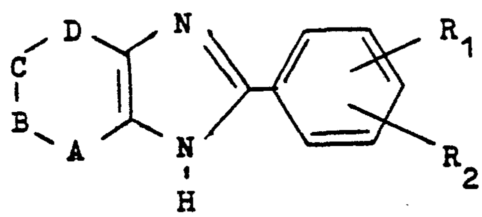

,(I)

in der

A, B, C und D je eine N-, HN-, $R_3N$-, O=C-, HC-, HO-C- oder $R_3SO_2$-O-C-Gruppe, wobei mindestens eine der Gruppen A, B, C oder D eine HN-Gruppe und eine andere der Gruppen A, B, C oder D eine O=C-Gruppe oder mindestens eine der Gruppen A, B, C oder D ein N-Atom und eine andere der Gruppen A, B, C oder D eine HO-C-, $R_3$O-C- oder eine $R_3SO_2$-O-C-Gruppe darstellen muß, oder auch einer der Reste A, B, C oder D eine Chlormethingruppe, wenn mindestens zwei der Reste A, B, C oder D ein N-Atom darstellen,

$R_1$ eine NC-$CH_2$-O-, HOOC-$CH_2$-O-, $R_3$-OOC-$CH_2$-O-, $R_3SO_2$-O-, $R_3$-$SO_2$-NH-, $R_3$-$SO_2$-$NR_3$- oder $R_4$-O-Gruppe oder, wenn A, B, C und D zusammen mit dem ankondensierten Imidazolring kein Imidazo[4,5-c]pyridin darstellen, eine Hydroxygruppe und

$R_2$ eine $R_3$O-, 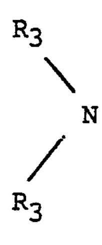 - oder $R_5$-O-Gruppe,

wobei $R_3$ jeweils eine Alkylgruppe mit 1 bis 4 Kohlenstoffatomen und

$R_4$ eine Alkinylgruppe mit 2 bis 5 Kohlenstoffatomen oder, wenn A, B, C und D zusammen mit dem ankondensierten Imidazolring kein Imidazo[4,5-c]pyridin bilden, auch eine Alkenylgruppe mit 2 bis 5 Kohlenstoffatomen und

$R_5$ eine Alkenyl- oder Alkinylgruppe mit jeweils 2 bis 5 Kohlenstoffatomen darstellen, bedeuten, deren Tautomere und deren Säureadditionssalze.

2. Imidazole der allgemeinen Formel I gemäß Anspruch 1, in der

A, B, C und D je eine N-, HN-, O=C-, HC-, HO-C- oder $R_3SO_2$-O-C-Gruppe, wobei mindestens eine der Gruppen A, B, C oder D eine HN-Gruppe und eine andere der Gruppen A, B, C oder D eine O=C-Gruppe oder mindestens eine der Gruppen A, B, C oder D ein N-Atom und eine andere der Gruppen A, B, C oder D eine HO-C- oder eine $R_3SO_2$-O-C-Gruppe darstellen muß,

$R_1$ eine NC-$CH_2$-O-, HOOC-$CH_2$-O-, $R_3$-OOC-$CH_2$-O-, $R_3SO_2$-O-, $R_3$-$SO_2$-NH-, $R_3$-$SO_2$-$NR_3$- oder $R_4$-O-Gruppe oder, wenn A, B, C und D zusammen mit dem ankondensierten Imidazolring kein Imidazo[4,5-c]pyridin darstellen, eine Hydroxygruppe, und

$R_2$ eine $R_3O$-, 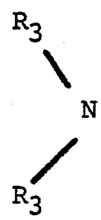 - oder $R_5$-O-Gruppe,

wobei $R_3$ jeweils eine Alkylgruppe mit 1 bis 3 Kohlenstoffatomen und

$R_4$ eine Alkinylgruppe mit 2 bis 5 Kohlenstoffatomen oder, wenn A, B, C und D zusammen mit dem ankondensierten Imidazolring kein Imidazo[4,5-c]pyridin bilden, eine Alkenylgruppe mit 2 bis 5 Kohlenstoffatomen und
$R_5$ eine Alkenyl- oder Alkinylgruppe mit jeweils 2 bis 5 Kohlenstoffatomen darstellen, bedeuten, deren Tautomere und deren Säureadditionssalze.

3. Imidazole der allgemeinen Formel I gemäß den Ansprüchen 1 und 2, in der
A bis D, $R_1$ und $R_2$ wie in den Ansprüchen 1 oder 2 definiert sind,
$R_3$ eine Methylgruppe und
$R_4$ eine Propargylgruppe oder, wenn A, B, C und D zusammen mit dem ankondensierten Imidazolring kein Imidazo-[4,5-c]pyridin bilden, eine Allylgruppe und
$R_5$ eine Allyl- oder Propargylgruppe bedeuten, deren Tautomere und deren Säureadditionssalze.

4. Imidazole der allgemeinen Formel I gemäß Anspruch 3, in denen $R_1$ in 4-Stellung und $R_2$ in 2-Stellung stehen, deren Tautomere und deren Säureadditionssalze.

5. Imidazo[4,5-b]pyridin-5-one, Imidazo[4,5-c]pyridin-4-one, Imidazo[4,5-c]pyridin-6-one, Imidazo[4,5-c]pyridazin-6-one, Imidazo[4,5-d]pyridazin-4-one, Imidazo[4,5-b]pyrazin-5-one, Purin-2-one, Purin-6-one, Purin-2,6-dione und Imidazo[4,5-e]triazin-6-one der allgemeinen Formel I gemäß Anspruch 2, in der
$R_1$ eine Propargyloxy-, Methansulfonyloxy-, Methansulfonylamino- oder N-Methyl-methansulfonylaminogruppe oder, wenn A, B, C und D zusammen mit dem ankondensierten Imidazolring kein Imidazo[4,5-c]pyridin bilden, auch eine Allyloxygruppe und
$R_2$ eine Methoxy- oder Dimethylaminogruppe bedeuten, deren Tautomere und deren Säureadditionssalze.

6. Imidazole der allgemeinen Formel I gemäß Anspruch 5, in der
$R_2$ wie im Anspruch 5 definiert ist und
$R_1$ eine Methansulfonyloxy-, Methansulfonylamino- oder N-Methyl-methansulfonylaminogruppe bedeutet, deren Tautomere und deren Säureadditionssalze.

7. 2-(2-Methoxy-4-methansulfonyloxy-phenyl)-5H-imidazo-[4,5-c]pyridin-4-on, dessen Tautomere und dessen Säureadditionssalze.

8. Physiologisch verträgliche Säureadditionssalze der Verbindungen der Ansprüche 1 bis 7 mit anorganischen oder organischen Säuren.

9. Arzneimittel, enthaltend eine Verbindung gemäß den Ansprüchen 1 bis 7 oder ein physiologisch verträgliches Säureadditionssalz hiervon neben einem oder mehreren inerten Trägerstoffen und/oder Verdünnungsmitteln.

10. Verfahren zur Herstellung der Verbindungen gemäß den Ansprüchen 1 bis 7 und von deren physiologisch verträglichen Säureadditionssalzen, dadurch gekennzeichnet, daß

a) eine gegebenenfalls im Reaktionsgemisch hergestellte Verbindung der allgemeinen Formel

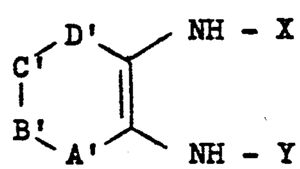

,(II)

in der
A' bis D' die für A bis D eingangs erwähnten Bedeutungen

besitzen oder einer der Reste A' bis D' eine durch eine Schutzgruppe geschützte Oxymethingruppe darstellt, einer der Reste X oder Y ein Wasserstoffatom und der andere der beiden Reste X und Y oder beide Reste X und Y eine Gruppe der Formel

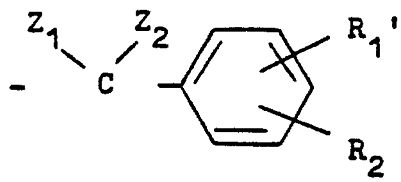

darstellen, in der

$R_2$ wie eingangs definiert ist, $R_1$' die für $R_1$ eingangs erwähnten Bedeutungen besitzt oder eine durch eine Schutzgruppe geschützte Hydroxygruppe darstellt, $Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert und anschließend eine gegebenenfalls verwendete Schutzgruppe abgespalten wird oder

b) ein N-Oxid der allgemeinen Formel

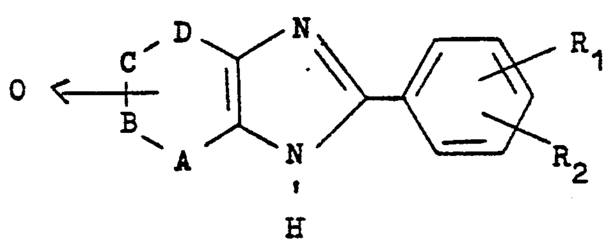

,(III)

in der
A, B, C, D, $R_1$ und $R_2$ wie eingangs definiert sind, umgelagert und gegebenenfalls anschließend hydrolysiert wird oder

c) zur Herstellung von Verbindungen der allgemeinen Formel I, in der $R_1$ eine Alkansulfonyloxy-, Alkansulfonylamino- oder N-Alkyl-alkansulfonylaminogruppe und/oder eine der Gruppen A, B, C oder D eine $R_3SO_2$-O-C-Gruppe darstellen, eine Verbindung der allgemeinen Formel

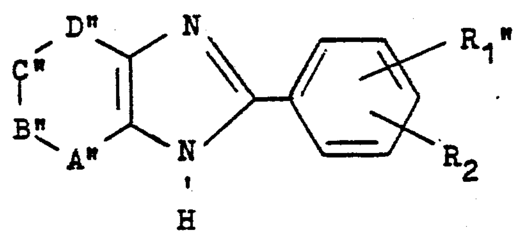
,(IV)

in der
$R_2$ wie eingangs definiert ist,
A", B", C", D" und $R_1$" die für A, B, C, D und $R_1$ eingangs erwähnten Bedeutungen besitzen, wobei jedoch entweder $R_1$" eine Hydroxy-, Amino- oder Alkylaminogruppe oder einer der Reste A", B", C" oder D" eine HO-C-Gruppe darstellen muß, mit einem Sulfonylhalogenid der allgemeinen Formel

$$Hal - SO_2 - R_3 \quad ,(V)$$

in der
$R_3$ wie eingangs definiert ist und
Hal ein Chlor- oder Bromatom darstellt, umgesetzt und anschließend gegebenenfalls partiell hydrolysiert wird oder

d) zur Herstellung von Verbindungen der allgemeinen Formel I, in der $R_1$ oder $R_1$ und $R_2$ eine Alkenyloxy- oder Alkinyloxygruppe oder $R_2$ eine Alkoxygruppe darstellen, eine Verbindung der allgemeinen Formel

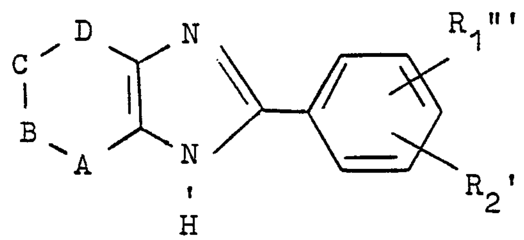

,(VI)

in der A, B, C und D wie eingangs definiert sind, $R_1$"' und $R_2$' die für $R_1$ und $R_2$ eingangs erwähnten Bedeutungen besitzen, wobei jedoch mindestens einer der Reste $R_1$"' oder $R_2$' eine Hydroxygruppe darstellen muß, mit einem Halogenid der allgemeinen Formel

$$Hal - R_6 \quad ,(VII)$$

in der $R_6$ die für $R_3$, $R_4$ und $R_5$ eingangs erwähnten Bedeutungen besitzt und Hal ein Chlor-, Brom- oder Jodatom darstellt, umgesetzt wird

und gewünschtenfalls anschließend eine so erhaltene Verbindung der allgemeinen Formel I in ihr Säureadditionssalz, insbesondere in ihr physiologisch verträgliches Säureadditionssalz mit einer anorganischen oder organischen Säure, übergeführt wird.

Patentansprüche (Benennungsland: AT)

1. Verfahren zur Herstellung von neuen Imidazolen der allgemeinen Formel

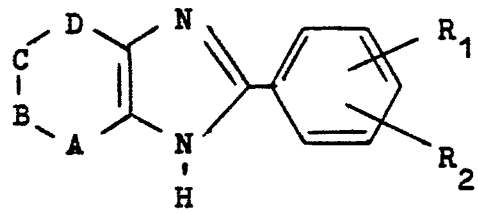

,(I)

in der

A, B, C und D je eine N-, HN-, $R_3$N-, O=C-, HC-, HO-C- oder $R_3SO_2$-O-C-Gruppe, wobei mindestens eine der Gruppen A, B, C oder D eine HN-Gruppe und eine andere der Gruppen A, B, C oder D eine O=C-Gruppe oder mindestens eine der Gruppen A, B, C oder D ein N-Atom und eine andere der Gruppen A, B, C oder D eine HO-C-, $R_3$O-C- oder eine $R_3SO_2$-O-C-Gruppe darstellen muß, oder auch einer der Reste A, B, C oder D eine Chlormethingruppe, wenn mindestens zwei der Reste A, B, C oder D ein N-Atom darstellen,

$R_1$ eine NC-$CH_2$-O-, HOOC-$CH_2$-O-, $R_3$-OOC-$CH_2$-O-, $R_3SO_2$-O-, $R_3$-$SO_2$-NH-, $R_3$-$SO_2$-$NR_3$- oder $R_4$-O-Gruppe oder, wenn A, B, C und D zusammen mit dem ankondensierten Imidazolring kein Imidazo[4,5-c]pyridin darstellen, eine Hydroxygruppe und

$R_2$ eine $R_3$O-, 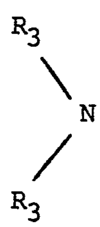 - oder $R_5$-O-Gruppe,

wobei $R_3$ jeweils eine Alkylgruppe mit 1 bis 4 Kohlenstoffatomen und

$R_4$ eine Alkinylgruppe mit 2 bis 5 Kohlenstoffatomen oder, wenn A, B, C und D zusammen mit dem ankondensierten Imidazolring kein Imidazo[4,5-c]pyridin bilden, auch eine Alkenylgruppe mit 2 bis 5 Kohlenstoffatomen und

$R_5$ eine Alkenyl- oder Alkinylgruppe mit jeweils 2 bis 5 Kohlenstoffatomen darstellen, bedeuten, von deren Tautomeren und von deren Säureadditionssalzen, insbesondere von deren physiologisch verträglichen Säureadditionssalzen, dadurch gekennzeichnet, daß

a) eine gegebenenfalls im Reaktionsgemisch hergestellte Verbindung der allgemeinen Formel

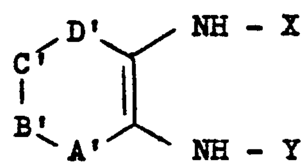

,(II)

in der

A' bis D' die für A bis D eingangs erwähnten Bedeutungen besitzen oder einer der Reste A' bis D' eine durch eine Schutzgruppe geschützte Oxymethingruppe darstellt, einer der Reste X oder Y ein Wasserstoffatom und der andere der beiden Reste X und Y oder beide Reste X und Y eine Gruppe der Formel

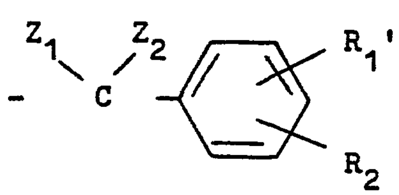

darstellen, in der

$R_2$ wie eingangs definiert ist, $R_1'$ die für $R_1$ eingangs erwähnten Bedeutungen besitzt oder eine durch eine Schutzgruppe geschützte Hydroxygruppe darstellt, $Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert und anschließend eine gegebenenfalls verwendete Schutzgruppe abgespalten wird oder

b) ein N-Oxid der allgemeinen Formel

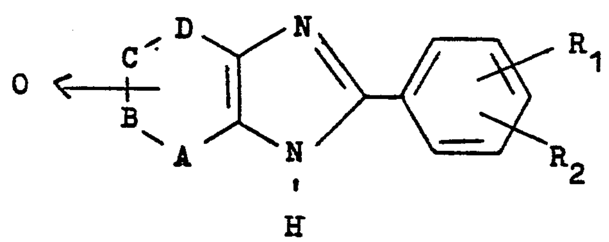
,(III)

in der A, B, C, D, $R_1$ und $R_2$ wie eingangs definiert sind, umgelagert und gegebenenfalls anschließend hydrolysiert wird oder

c) zur Herstellung von Verbindungen der allgemeinen Formel I, in der $R_1$ eine Alkansulfonyloxy-, Alkansulfonylamino- oder N-Alkyl-alkansulfonylaminogruppe und/oder eine der Gruppen A, B, C oder D eine $R_3SO_2$-O-C-Gruppe darstellen, eine Verbindung der allgemeinen Formel

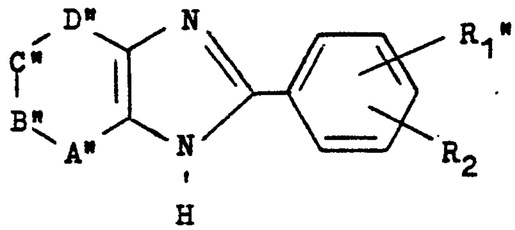

,(IV)

in der

$R_2$ wie eingangs definiert ist,

A", B", C", D" und $R_1$" die für A, B, C, D und $R_1$ eingangs erwähnten Bedeutungen besitzen, wobei jedoch entweder $R_1$" eine Hydroxy-, Amino- oder Alkylaminogruppe oder einer der Reste A", B", C" oder D" eine HO-C-Gruppe darstellen muß, mit einem Sulfonylhalogenid der allgemeinen Formel

$$Hal - SO_2 - R_3 \quad ,(V)$$

in der

$R_3$ wie eingangs definiert ist und

Hal ein Chlor- oder Bromatom darstellt, umgesetzt und anschließend gegebenenfalls partiell hydrolysiert wird oder

d) zur Herstellung von Verbindungen der allgemeinen Formel I, in der $R_1$ oder $R_1$ und $R_2$ eine Alkenyloxy- oder Alkinyloxygruppe oder $R_2$ eine Alkoxygruppe darstellen, eine Verbindung der allgemeinen Formel

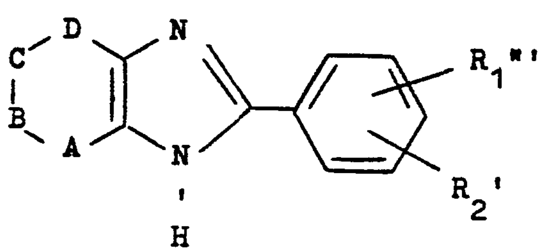

,(VI)

in der
A, B, C und D wie eingangs definiert sind,
$R_1$"' und $R_2$' die für $R_1$ und $R_2$ eingangs erwähnten Bedeutungen besitzen, wobei jedoch mindestens einer der Reste $R_1$"' oder $R_2$' eine Hydroxygruppe darstellen muß, mit einem Halogenid der allgemeinen Formel

$$Hal - R_6 \qquad ,(VII)$$

in der
$R_6$ die für $R_3$, $R_4$ und $R_5$ eingangs erwähnten Bedeutungen besitzt und
Hal ein Chlor-, Brom- oder Jodatom darstellt, umgesetzt wird

und gewünschtenfalls anschließend eine so erhaltene Verbindung der allgemeinen Formel I in ihr Säureadditionssalz, insbesondere in ihr physiologisch verträgliches Säureadditionssalz mit einer anorganischen oder organischen Säure, übergeführt wird.

2. Verfahren gemäß Anspruch 1 zur Herstellung von Verbindungen der allgemeinen Formel I gemäß Anspruch 1, in der
$R_1$ und $R_2$ wie im Anspruch 1 definiert sind,
A, B, C und D je eine N-, HN-, O=C-, HC-, HO-C- oder $R_3SO_2$-O-C-Gruppe, wobei mindestens eine der Gruppen A, B, C oder D eine HN-Gruppe und eine andere der Gruppen A, B, C oder D eine O=C-Gruppe oder
mindestens eine der Gruppen A, B, C oder D ein N-Atom und eine andere der Gruppen A, B, C oder D eine HO-C- oder eine $R_3SO_2$-O-C-Gruppe darstellen muß, bedeuten, von deren Tautomeren und von deren Säureadditionssalzen, insbesondere von deren physiologisch verträglichen Säureadditionssalzen, dadurch gekennzeichnet, daß

0130461

a) eine gegebenenfalls im Reaktionsgemisch hergestellte Verbindung der allgemeinen Formel

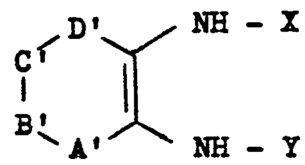

,(II)

in der
A' bis D' die für A bis D eingangs erwähnten Bedeutungen besitzen oder einer der Reste A' bis D' eine durch eine Schutzgruppe geschützte Oxymethingruppe darstellt, einer der Reste X oder Y ein Wasserstoffatom und der andere der beiden Reste X und Y oder beide Reste X und Y eine Gruppe der Formel

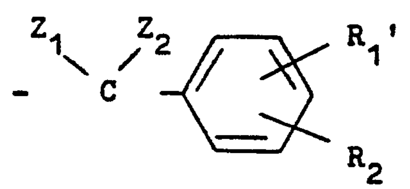

darstellen, in der

$R_1'$, $R_2$, $Z_1$ und $Z_2$ wie im Anspruch 1 definiert sind, cyclisiert und anschließend eine gegebenenfalls verwendete Schutzgruppe abgespalten wird oder

b) ein N-Oxid der allgemeinen Formel

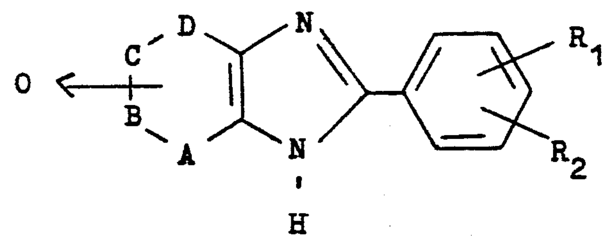

,(III)

in der
A, B, C und D wie eingangs und $R_1$ und $R_2$ wie im Anspruch 1 definiert sind, umgelagert und gegebenenfalls anschließend hydrolysiert wird oder

c) zur Herstellung von Verbindungen der allgemeinen Formel I, in der $R_1$ eine Alkansulfonyloxy-, Alkansulfonylamino- oder N-Alkyl-alkansulfonylaminogruppe und/oder eine der Gruppen A, B, C oder D eine $R_3SO_2$-O-C-Gruppe darstellen, eine Verbindung der allgemeinen Formel

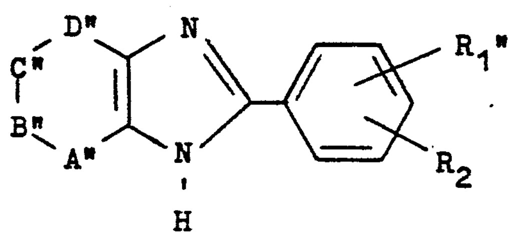

,(IV)

in der
$R_2$ wie im Anspruch 1 definiert ist,
A", B", C" und D" wie eingangs und die für A, B, C und D eingangs erwähnten Bedeutungen besitzen und
$R_1$' die für $R_1$ im Anspruch 1 erwähnten Bedeutungen besitzt, wobei jedoch entweder $R_1$" eine Hydroxy-,

Amino- oder Alkylaminogruppe oder einer der Reste A", B", C" oder D" eine HO-C-Gruppe darstellen muß, mit einem Sulfonylhalogenid der allgemeinen Formel

$$Hal - SO_2 - R_3 \qquad ,(V)$$

in der $R_3$ und Hal wie im Anspruch 1 definiert sind, umgesetzt und anschließend gegebenenfalls partiell hydrolysiert wird oder

d) zur Herstellung von Verbindungen der allgemeinen Formel I, in der $R_1$ oder $R_1$ und $R_2$ eine Alkenyloxy- oder Alkinyloxygruppe oder $R_2$ eine Alkoxygruppe darstellen, eine Verbindung der allgemeinen Formel

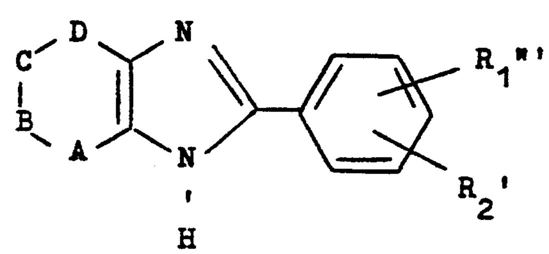

,(VI)

in der A, B, C und D wie eingangs definiert sind, $R_1$"' und $R_2$' die für $R_1$ und $R_2$ im Anspruch 1 erwähnten Bedeutungen besitzen, wobei jedoch mindestens einer der Reste $R_1$"' oder $R_2$' eine Hydroxygruppe darstellen muß, mit einem Halogenid der allgemeinen Formel

$$Hal - R_6 \qquad ,(VII)$$

in der $R_6$ die für $R_3$, $R_4$ und $R_5$ im Anspruch 1 erwähnten Bedeutungen besitzt und Hal wie im Anspruch 1 definiert ist, umgesetzt wird

und gewünschtenfalls anschließend eine so erhaltene Verbindung der allgemeinen Formel I in ihr Säureadditionssalz, insbesondere in ihr physiologisch verträgliches Säureadditionssalz mit einer anorganischen oder organischen Säure, übergeführt wird.

3. Verfahren gemäß den Ansprüchen 1 und 2, dadurch gekennzeichnet, daß die Umsetzung in einem Lösungsmittel durchgeführt wird.

4. Verfahren gemäß den Ansprüchen 1a, 2a und 3, dadurch gekennzeichnet, daß die Umsetzung in Gegenwart eines Acylierungsmittels oder Kondensationsmittels durchgeführt wird.

5. Verfahren gemäß den Ansprüchen 1a, 2a, 3 und 4, dadurch gekennzeichnet, daß die Umsetzung bei Temperaturen zwischen 0 und 250°C, vorzugsweise jedoch bei der Siedetemperatur des Reaktionsgemisches, durchgeführt wird.

6. Verfahren gemäß den Ansprüchen 1a, 2a und 3 bis 5, dadurch gekennzeichnet, daß die nachträgliche Abspaltung einer verwendeten Schutzgruppe hydrolytisch oder hydrogenolytisch erfolgt.

7. Verfahren gemäß Anspruch 1b, 2b und 3, dadurch gekennzeichnet, daß die Umsetzung in Gegenwart eines Acylierungsmittels erfolgt.

8. Verfahren gemäß den Ansprüchen 1b, 2b, 3 und 7, dadurch gekennzeichnet, daß die Umsetzung bei erhöhten Temperaturen, vorzugsweise jedoch bei der Siedetemperatur des Reaktionsgemisches, durchgeführt wird.

9. Verfahren gemäß den Ansprüchen 1b, 2b, 3, 7 und 8, dadurch gekennzeichnet, daß die anschließende Hydrolyse in Gegenwart einer Säure oder Base durchgeführt wird.

10. Verfahren gemäß den Ansprüchen 1c, 1d und 3, dadurch gekennzeichnet, daß die Umsetzung in Gegenwart eines säurebindenden Mittels und bei Temperaturen zwischen 0 und 100°C, vorzugsweise jedoch bei Temperaturen zwischen 20 und 50°C, durchgeführt wird.